Europäisches Patentamt

European Patent Office

Office européen des brevets

(19)

(11) **EP 1 214 279 B1**

(12) **EUROPÄISCHE PATENTSCHRIFT**

(45) Veröffentlichungstag und Bekanntmachung des Hinweises auf die Patenterteilung:
**26.05.2004 Patentblatt 2004/22**

(21) Anmeldenummer: **00966000.2**

(22) Anmeldetag: **14.09.2000**

(51) Int Cl.$^7$: **C07C 17/02**, C07C 19/045

(86) Internationale Anmeldenummer:
**PCT/EP2000/008963**

(87) Internationale Veröffentlichungsnummer:
**WO 2001/021564 (29.03.2001 Gazette 2001/13)**

(54) **VERFAHREN ZUR WÄRMENUTZUNG BEI DER HERSTELLUNG VON 1,2-DICHLORETHAN**

METHOD FOR UTILIZING HEAT IN THE PRODUCTION OF 1,2-DICHLOROETHANE

PROCEDE PERMETTANT D'UTILISER LA CHALEUR PRODUITE LORS DE LA FABRICATION DU 1,2-DICHLORETHANE

(84) Benannte Vertragsstaaten:
**AT BE CH CY DE DK ES FI FR GB GR IE IT LI LU MC NL PT SE**

(30) Priorität: **22.09.1999 DE 19945355**
**29.07.2000 DE 10037323**

(43) Veröffentlichungstag der Anmeldung:
**19.06.2002 Patentblatt 2002/25**

(73) Patentinhaber: **Uhde GmbH**
**44141 Dortmund (DE)**

(72) Erfinder: **MOTZ, Joachim**
**65779 Kelkheim (DE)**

(56) Entgegenhaltungen:
**EP-A- 0 131 932      DE-A- 19 641 562**
**FR-A- 2 578 537      US-A- 4 873 384**

**Beschreibung**

Die Erfindung richtet sich auf ein Verfahren zur Herstellung von 1,2-Dichlorethan, im Folgenden als "EDC" bezeichnet, welches überwiegend als Zwischenprodukt der Herstellung von monomerem Vinylchlorid, im Folgenden als "VCM" bezeichnet, dient, woraus letztlich Polyvinylchlorid, PVC, hergestellt wird.

Bei der Umsetzung von EDC zu VCM entsteht Chlorwasserstoff (HCl), EDC wird daher bevorzugt aus Ethen ($C_2H_4$) und Chlor ($Cl_2$) derart hergestellt, dass hinsichtlich des bei den Umsetzungen erzeugten und verbrauchten Chlorwasserstoffes (HCl) eine ausgewogene Bilanz entsprechend den folgenden Reaktionsgleichungen erreicht wird:

$$Cl_2 + C_2H_4 \rightarrow C_2H_4Cl_2 \text{ (EDC)} + 180 \text{ kJ/Mol} \tag{1}$$

$$C_2H_4Cl_2 \text{ (EDC)} \rightarrow C_2H_3Cl \text{ (VCM)} + HCl - 71 \text{ kJ/Mol} \tag{2}$$

$$C_2H_4 + 2 \text{ HCl} + \tfrac{1}{2} O_2 \rightarrow C_2H_4Cl_2 \text{ (EDC)} + H_2O + 238 \text{ kJ/Mol} \tag{3}$$

Der Teil des Herstellungsverfahrens, der sich auf die Reaktion (1) bezieht, wird im Folgenden als "Direktchlorierung", der Teil, der sich auf Reaktion (2) bezieht, als EDC-Spaltung und der Teil, der sich auf Reaktion (3) bezieht, als Oxihydrochlorierung bezeichnet. Das gesamte Herstellungsverfahren, sofern das in der EDC-Spaltung erzeugte HCl vollständig in der Oxihydrochlorierung verbraucht wird, wird im Folgenden als "ausgewogenes Verfahren" bezeichnet.

Die Direktchlorierung geschieht dabei üblicherweise in einem Schlaufenreaktor, für den unterschiedliche Ausführungsformen bekannt sind. Ein solches Verfahren ist beispielsweise in der Schrift DE 43 18 609 A1 beschrieben. Es zeigt sich dabei seit vielen Jahren, dass der Reinheit des dort erzeugten EDC hinsichtlich der Wirtschaftlichkeit und der letztendlich erzielbaren Produktreinheit bezüglich des VCM der Reaktion (2) und somit des Gesamtverfahrens eine überragende Bedeutung zukommt. Daher wurden in der Vergangenheit viele Versuche unternommen, die Nebenreaktionen von Reaktion (1) zu minimieren.

Ein besonders erfolgreicher Versuch war die Absenkung der Reaktionstemperatur, nachteilig hierbei war jedoch, dass die Reaktionswärme dann bei entsprechend niedrigerer Temperatur anfiel und keine sinnvolle Nutzungsmöglichkeit mehr innerhalb der EDC-Herstellung bestand. Die Reaktionswärme wurde so zu Abwärme, was dazu führte, dass diese Möglichkeit der Minimierung von Nebenreaktionen der Reaktion (1) von den Fachleuten verworfen wurde.

Den Wärmeverschiebungsmaßnahmen beim ausgewogenen Verfahren kommt große wirtschaftliche und ökologische Bedeutung zu. Beispielsweise wird in der Schrift DE 41 31 576 A1 ein aufwendiges Nutzungsverfahren für die Wärme von EDC-Brüden beschrieben. Der für eine ausgewogene Bilanz nötige Bedarf an EDC wird gemäß Reaktion (1) bei Temperaturen von 75 bis 100 °C unter Sieden des EDC, Kondensieren der Brüden und Rücklauf des um den Produktstrom reduzierten kondensierten Brüdenstroms zum Reaktor hergestellt. Auch die Auskopplung von Wärme aus flüssigem, im Reaktor umlaufenden EDC wurde schon durchgeführt. Die zur Kondensation der aus dem EDC-Reaktor stammenden Brüden sowie ggf. darin umlaufenden flüssigen EDC notwendige Kühlleistung liegt bei 420 kWh pro erzeugter Tonne VCM. Liegt Chlor als Einsatzprodukt flüssig vor, so beträgt die notwendige Heizleistung zum Verdampfen des Chlors 35 kWh pro erzeugter Tonne VCM.

Sowohl das EDC aus der Oxihydrochlorierung und gegebenenfalls auch aus der Direktchlorierung als auch das nicht umgesetzte EDC aus der EDC-Spaltung müssen destillativ aufbereitet werden, da die EDC-Spaltung ein hochreines EDC als Einsatz erfordert. Zur Destillation werden üblicherweise Destillationskolonnen verwendet, zur Abtrennung von Wasser und Leichtsiedern wird eine Entwässerungs- bzw. Leichtsiederkolonne und zur Abtrennung von Hochsiedern eine oder zwei Kolonnen verwendet, die als Hochsiederkolonne und als Vakuumkolonne bezeichnet werden. Die zum Betrieb dieser Destillationen notwendige Dampfmenge liegt bei etwa 513 kWh pro Tonne erzeugtem VCM, wenn davon ausgegangen wird, dass das EDC aus der Direktchlorierung, dasjenige aus der Oxihydrochlorierung sowie das nicht umgesetzte Rest-EDC aus der Spaltung gemeinsam gereinigt werden. Ist eine Destillation des EDC aus der Direktchlorierung nicht nötig, weil die Reaktionsbedingungen so eingestellt werden können, dass die erforderliche Reinheit für die EDC-Spaltung auch ohne Destillation erreicht werden kann, reduziert sich die für die Reinigung des restlichen EDC notwendige Dampfmenge auf 385 kWh pro Tonne erzeugtes VCM.

Es sind schon Verfahren vorgeschlagen worden, die gesamte Reaktionswärme der Direktchlorierung durch Kondensation der Brüden aus der Direktchlorierung direkt unter Beheizung der Hochsiederkolonne und Vakuumkolonne zu nutzen. Wegen der dann dafür notwendigen, relativ hohen Reaktionstemperatur in der Direktchlorierung reduzieren derartige Verfahren jedoch die Ausbeute bezogen auf die Einsatzstoffe Chlor und Ethen und bedingen weiterhin eine schlechtere Qualität des EDC aus der Direktchlorierung, was vermehrte destillative Aufbereitung erfordert und somit zu einer Reduzierung der Wirtschaftlichkeit führt.

Die US 4,873,384 und die DE-A 19641562 beschreiben Verfahren zur Direktchlorierung, bei denen das dampfförmig aus Direktchlorierungsreaktoren gewonnene EDC zur Beheizung von Kolonnen verwendet wird. Um die Wärme bei der hierfür erforderlichen Temperatur bereitstellen zu können, wird der Direktchlorierungsreaktor im Verfahren der US 4,873,384 zwischen 83 und 160 °C, beispielsweise bei 135 °C, betrieben, im Verfahren der DE-A 19641562 kann die Reaktortemperatur zwischen 65 und 125 °C gewählt werden. In beiden Fällen muss allerdings der Druck der zu beheizenden Kolonne entsprechend eingestellt, in der Regel also abgesenkt werden.

Verfahren, die mittels Brüdenverdichtung einen wirtschaftlicheren Betrieb von Kolonnen zu erreichen versuchen, sind bereits beschrieben worden. So stellt die Schrift FR 2 578 537 A1 ein Verfahren vor, mittels Brüdenverdichtung einer EDC-Hochsiederabtrennkolonne deren eigene Sumpfbeheizung zu bewirken.

Die Schrift EP 0 131 932 A1 stellt ein Verfahren vor, mit dem mittels Brüdenverdichtung einer EDC-Hochsiederabtrennkolonne deren eigene Sumpfbeheizung und/oder die Beheizung einer Leichtsiederkolonne betrieben werden kann.

Die bereits oben erwähnte Schrift DE 41 31 576 A1 stellt ein Verfahren vor, die Brüden der Hochsiederabtrennkolonne dazu zu nutzen, die Sumpfbeheizung der Entwässerungskolonne zu betreiben.

Aus der Schrift EP 0 180 925 B1 ist weiterhin ein Verfahren bekannt, bei der Brüden einer Hochsiederabtrennkolonne einer VCM-Herstellung verdichtet und dazu genutzt wird, den Sumpf der Kolonne zu beheizen. Diese Schrift lehrt auch, dass geprüft werden muss, ob während des Verdichtungsvorgangs weitere Reaktionen ablaufen, die zu Verunreinigungen im EDC sowie zu betrieblichen Problemen des Verdichters führen können.

Im Unterschied zur vorliegenden Erfindung benutzen die oben erwähnten Technologien die Brüden von Reinigungskolonnen und nicht den Brüden des Direktchlorierungsreaktors.

Daraus resultierend ist es die Aufgabe der vorliegenden Erfindung, trotz niedriger Reaktionstemperatur in der Direktchlorierung die dort anfallende Reaktionswärme zu nutzen.

Diese Aufgabe wird mit einem Verfahren der eingangs bezeichneten Art gemäß der Erfindung dadurch gelöst, dass, wie im Anspruch 1 angegeben, das dampfförmig aus dem Reaktionsreaktor der Direktchlorierung gewonnene EDC verdichtet wird und dieses verdichtete EDC zur Wärmeabgabe Wärmetauschern zugeführt wird.

Es zeigt sich, dass durch diese Maßnahme die Reaktionstemperatur in der Direktchlorierung soweit abgesenkt werden kann, dass das in der Direktchlorierung erzeugte EDC ohne weitere destillative Reinigung in die EDC-Spaltung geführt werden kann, ohne dass damit der Nachteil verbunden ist, dass die Reaktionswärme bei für eine Verwertung zu niedriger Temperatur anfällt. Durch die Verdichtung steigen nämlich sowohl die Temperatur des verdichteten EDC als auch die Kondensationstemperatur des EDC, wodurch es möglich wird, das verdichtete EDC bei höherer Temperatur als der der Direktchlorierung einzusetzen.

Weitere Ausgestaltungen der Erfindung ergeben sich aus den Unteransprüchen. So ist insbesondere vorgesehen, dass die verdichteten Brüden dem Verdampfer einer Leichtsieder- bzw. Entwässerungskolonne und/oder dem Verdampfer einer Hochsiederkolonne und/oder dem Verdampfer einer Vakuumkolonne und/oder dem Chlorverdampfer vor dem Direktchlorierungsreaktor zugeführt werden. Da es ein Vorteil der Erfindung ist, dass die in der Direktchlorierung entstehende Reaktionswärme von 420 kWh pro Tonne erzeugtes VCM ziemlich genau der Summe des Bedarfs der für den Betrieb der Destillationskolonnen von 385 kWh pro Tonne erzeugtes VCM und der Chlorverdampfung von 35 kWh pro Tonne erzeugtes VCM benötigten Heizwärme entspricht, gibt die vorliegende Erfindung dem Fachmann das Mittel in die Hand, die Reaktionswärme auf die genannten Verbraucher zu verteilen und den Einsatz von Fremdenergie auf den Bedarf für die Anlagenregelung und den Betrieb der erfindungswesentlichen Verdichtung beschränken zu können.

Mit der Erfindung wird eine kompakte Bauweise der erfindungsgemäßen Vorrichtung möglich, da mit den komprimierten Brüden eine Beheizung der EDC-Kolonne ebenso möglich ist wie die Beheizung weiterer Kolonnen, wie dies in Ausgestaltung nach der Erfindung ebenfalls vorgesehen ist. Hierbei wird für die Verdichtung des dampfförmig aus dem Direktchlorierungsreaktor abgezogenen 1,2-Dichlorethans ein Turboverdichter eingesetzt. In einer weiteren Ausgestaltung der Erfindung wird der Turboverdichter mit einer Wellenabdichtung in Tandembauweise ausgestattet und diese Wellenabdichtung mit Stickstoff als Sperrgas beaufschlagt. In einer weiteren Ausgestaltung der Erfindung wird die Fördermenge des Turboverdichters mittels Drehzahlregelung an die Ausbringung des Direktchlorierungsreaktors angepasst.

Weitere Merkmale, Einzelheiten und Vorteile der Erfindung ergeben sich aufgrund der nachfolgenden Beschreibung sowie anhand der Zeichnung.

Diese zeigt in

Figur 1     ein Beispiel einer Anlagenschaltung nach der Erfindung und in
Figur 2     die Schaltung einer Versuchsanordnung zu einem Funktionstest.

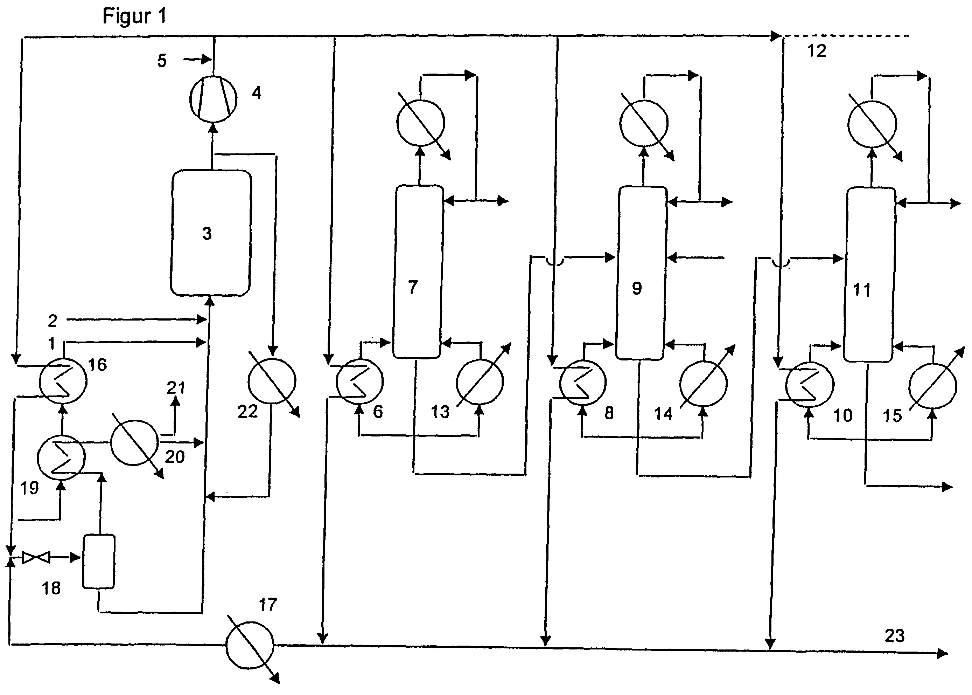

Im Beispiel gemäß Figur 1 reagieren verdampftes Chlorgas 1 und Ethen 2 im Direktchlorierungsreaktor 3 zu EDC. Die Reaktionswärme wird durch verdampfendes EDC (Brüden) mit den Brüden aus dem Direktchlorierungsreaktor 3 ausgeschleust. Im Verdichter 4 wird der Brüden komprimiert. Den komprimierten Brüden wird die Überhitzungswärme durch Einspritzen von flüssigem EDC 5 entzogen. Der komprimierte und gesättigte Brüden beheizt den Verdampfer 6 der Leichtsieder-Entwässerungskolonne 7, den Verdampfer 8 der Hochsiederkolonne 9, den Verdampfer 10 der Vakuumkolonne 11 und/oder weitere Wärmetauscher 12. Für Anfahrzwecke oder instabile Betriebszustände können alle Kolonnen auch mit Sattdampf 13, 14, 15 beheizt werden. Der verdichtete Brüden kann ebenfalls zur Überhitzung des verdampften Chlors 16 benutzt werden.

Durch entsprechende Kühlung mittels des Trimmkühlers 17 und Entspannung 18 der gesamten kondensierten Brüden entsteht genau die EDC-Flashdampfmenge, die durch Kondensation zum Beheizen des Chlorverdampfers 19 nötig ist. Nicht kondensierter EDC-Flashdampf wird in einer Kühlfalle 20 bis auf einen Rest weiter kondensiert, der überwiegend aus Inertgasen besteht und einer Ausschleusung 21 zugeführt wird. Auf diesem Wege wird auch die über die Wellenabdichtung des Turboverdichters dem EDC-Brüden als Verunreinigung beigemischte Sperrgasteilmenge wieder vom EDC abgetrennt.

Für Anfahrzwecke oder instabile Betriebszustände kann der Direktchlorierungsreaktor auch mit Kühlwasser 22 gekühlt werden. Der Trimmkühler 17 sorgt für die ausgeglichene Wärmebilanz. Die Stoffbilanz wird durch Abzug des Produkt-EDC 23 vom auskondensierten Brüden eingehalten.

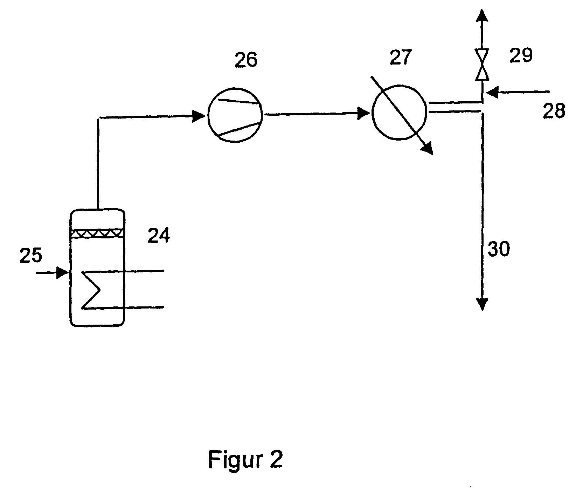

Anhand der Figur 2 wird ein Funktionstest beschrieben, wie ihn auch die Schrift EP 0 180 925 B1 mitteilt, wobei die Temperaturen in Celsiusgraden und die Druckangaben in Absolutdrücken angegeben sind. Gew.-ppm bedeuten mg der jeweiligen Substanz pro kg Gesamtgewicht der EDC-Fraktion: Es wurde EDC der folgenden Zusammensetzung verwendet, wie sie aus dem Brüden eines Direktchlorierungsreaktors mit abgesenkter Reaktionstemperatur erhalten wird:

| | |
|---|---|
| 99,9 | Gew.-% EDC (1,2-Dichlorethan) |
| 260 | Gew.-ppm 1,1-Dichlorethan |
| 490 | Gew.-ppm 1,1,2-Trichlorethan |
| 140 | Gew.-ppm Chlorwasserstoff |
| 110 | Gew.-ppm Ethen |

In einem beheizten Behälter 24 wurde dieses EDC verdampft, wobei der Stand im Behälter 24 durch Zufuhr 25 von EDC gleicher Zusammensetzung konstant gehalten wurde. Die Verdampfung erfolgt bei einem Druck von 1,3 bar und der zugehörigen Sattdampftemperatur von 92 °C. Die Brüden wurden von einem Verdichter 26 auf einen Druck von 3,05 bar bei einer Temperatur von 133°C verdichtet. Die um ca. 8 K überhitzten Brüden wurden im Kondensator 27 auskondensiert. Zur Druckhaltung 29 erfolgte nach dem Kondensator 27 eine Überlagerung mit Stickstoff 28. Die auskondensierten Brüden 30 haben genau die gleiche Zusammensetzung wie die unverdichteten Brüden, beim Vergleich der Analysen vor und nach dem Verdichter ist kein Unterschied feststellbar.

Daraus folgt, dass das aus dem bei abgesenkter Temperatur betriebenen Direktchlorierungsreaktor hochrein erhaltene EDC eine noch geringere Neigung zu Nachreaktionen und den damit verbundenen betrieblichen Problemen des Verdichters besitzt als jenes destillativ gereinigte EDC aus dem Kopf der Destillationskolonnen entsprechend dem aus der Schrift EP 0 180 925 B1 bekannten Stand der Technik, womit die vorliegende Erfindung einen weiteren Vorteil aufweisen kann.

**Patentansprüche**

1.     Verfahren zur Wärmenutzung bei der Herstellung von 1,2-Dichlorethan mittels Direktchlorierung aus Chlor und Ethen, **dadurch gekennzeichnet, dass** das dampfförmig aus dem Direktchlorierungsreaktor (3) gewonnene 1,2-Dichlorethan verdichtet wird und dieses verdichtete 1,2-Dichlorethan zur Wärmeabgabe dem Verdampfer (6) einer Leichtsieder-Entwässerungskolonne (7) und/oder dem Verdampfer (8) einer Hochsiederkolonne (9) und/oder dem Verdampfer (10) einer Vakuumkolonne (11) und/oder dem Chlorerhitzer (16) vor dem Direktchlorierungsreaktor (3) zugeführt wird.

2.     Verfahren nach Anspruch 1 oder 2, **dadurch gekennzeichnet, dass** die Beheizung der mit einem Kopfdruck von 1,0 bis 1,6 bar absolut betriebenen Entwässerungskolonne (7) zur Reinigung des 1,2-Dichlorethans durch Wärmetausch zwischen ihrem Sumpfprodukt und den verdichteten Brüden aus der Direktchlorierung bei einer Temperaturdifferenz von 8 bis 25 °C vorgenommen wird, wobei die Sumpftemperatur durch entsprechenden Blasenabzug bei 80 bis 105 °C gehalten wird.

**3.** Verfahren nach einem der vorangehenden Ansprüche, **dadurch gekennzeichnet, dass** die Beheizung der mit einem Kopfdruck von 0,7 bis 1,4 bar absolut betriebenen Hochsiederkolonne (9) zur Reinigung des nicht gespaltenen 1,2-Dichlorethans und des 1,2-Dichlorethans aus der Entwässerungskolonne (7) durch Wärmetausch zwischen dem Sumpfprodukt und den verdichteten Brüden aus der Direktchlorierung bei einer Temperaturdifferenz von 8 bis 25 °C vorgenommen wird und die Sumpftemperatur durch Blasenabzug bei 84 bis 105 °C gehalten wird.

**4.** Verfahren nach einem der vorangehenden Ansprüche, **dadurch gekennzeichnet, dass** die Beheizung der mit einem Kopfdruck von 0,2 bis 0,3 bar absolut betriebenen Vakuum-Kolonne (11) zur Reinigung des Blasenabzuges aus der Hochsiederkolonne (9) durch Wärmetausch zwischen ihrem Sumpfprodukt und den verdichteten Brüden aus der Direktchlorierung bei einer Temperaturdifferenz von 8 bis 25 °C vorgenommen wird und die Sumpftemperatur durch Blasenabzug bei 80 bis 90 °C gehalten wird.

**5.** Verfahren nach einem der vorangehenden Ansprüche, **dadurch gekennzeichnet, dass** flüssiges Chlor zur Direktchlorierung durch Wärmetausch mit den verdichteten Brüden aus der Direktchlorierung oder durch Wärmetausch mit zirkulierendem, flüssigen 1,2-Dichlorethan aus der Direktchlorierung verdampft und überhitzt wird.

**6.** Verwendung eines Turboverdichters für die Verdichtung des dampfförmig aus dem Direktchlorierungsreaktor (3) abgezogenen 1,2-Dichlorethans für das Verfahren gemäß einem der vorangehenden Ansprüche.

**7.** Verwendung eines Turboverdichters gemäß Anspruch 6 aufweisend eine Wellenabdichtung in Tandembauweise, wobei die Wetlenabdichtung mit Stickstoff als Sperrgas beaufschlagt wird.

**8.** Verwendung gemäß einem der Ansprüche 6 oder 7, **dadurch gekennzeichnet, dass** die Fördermenge des Turboverdichters mittels Drehzahlregelung an die Ausbringung des Direktchlorierungsreaktors angepasst wird.

**Claims**

**1.** Process for heat recovery in the production of 1,2-dichloroethane from chlorine and ethene by direct chlorination, **characterised in that** the vaporous 1,2-dichloroethane obtained from direct chlorination reactor (3) is compressed and for heat recovery, the compressed 1,2-dichloroethane is fed to evaporator (6) of a light ends dehydration column (7) and/or to evaporator (8) of a heavy ends column (9) and/or to evaporator (10) of a vacuum column (11) and/or to chlorine heater (16) upstream of direct chlorination reactor (3).

**2.** Process according to claim 1, **characterised in that** the dehydration column (7) for purifying 1,2-dichloroethane, which is operated at a head pressure of 1.0 to 1.6 bars abs., is heated by providing heat exchange between the bottom product and the compressed vapours from the direct chlorination reactor at a temperature difference in the range of 8 to 25°C, the bottom temperature being maintained in the range of 80 to 105°C by an adequate bottom discharge stream.

**3.** Process according to any of the preceding claims, **characterised in that** the heavy ends column (9) for purifying the non-cracked 1,2-dichloroethane and the 1,2-dichloroethane from the dehydration column (7), which is operated at a head pressure of 0.7 to 1.4 bars abs., is heated by providing heat exchange between the bottom product and the compressed vapours from the direct chlorination reactor at a temperature difference in the range of 8 to 25°C, the bottom temperature being maintained in the range of 84 to 105°C by an adequate bottom discharge stream.

**4.** Process according to any of the preceding claims, **characterised in that** the vacuum column (11) used for purifying the bottom discharge stream from the heavy ends column (9) and operated at a head pressure of 0.2 to 0.3 bars abs. is heated by providing heat exchange between the bottom product and the compressed vapours from the direct chlorination reactor at a temperature difference in the range of 8 to 25°C, the bottom temperature being maintained in the range of 80 to 90 °C by an adequate bottom discharge stream.

**5.** Process according to one of the preceding claims, **characterised in that** liquid chlorine used for direct chlorination is evaporated and superheated by heat exchange with the compressed vapours from the direct chlorination reactor or by heat exchange with circulated liquid 1,2-dichloroethane from the direct chlorination reactor.

**6.** Use of a turbocompressor in the process according to any of the preceding claims for compressing the vapourous 1,2-dichloroethane withdrawn from the direct chlorination reactor (3).

**7.** Use of a turbocompressor according to claim 6, which is equipped with a tandem type shaft seal and in that a device is provided for supplying said shaft seal with nitrogen as barrier gas.

**8.** Use of a turbocompressor according to claim 6 or 7,
**characterised in that** a speed controller is provided to adjust the delivery rate of the turbo-compressor to the discharge rate of the direct chlorination reactor.


**Revendications**

**1.** Procédé pour la récupération de chaleur pendant la production de 1,2-dichloréthane à partir du chlore et de l'éthène moyennant la chlorination directe,
**caractérisé en ce que** le 1,2-dichloréthane obtenu à l'état de vapeur dans le réacteur de chlorination directe (3) est comprimé et que pour la récupération de la chaleur, le 1,2-dichloréthane comprimé est introduit dans l'évaporateur (6) de la colonne de déshydratation de la fraction à bas point d'ébullition (7) et/ou dans l'évaporateur (8) d'une colonne à fraction de haut point d'ébullition et/ou dans l'évaporateur (10) d'une colonne à vide (11) et/ou dans le réchauffeur de chlore (16) monté en amont du réacteur de chlorination directe (3).

**2.** Procédé selon la revendication 1,
**caractérisé en ce que** la colonne de déshydratation (7) qui fonctionne à une pression de tête de 1,0 à 1,6 bars abs. est chauffée pour assurer la purification du 1,2-dichloréthane, le chauffage ayant lieu par un échange de chaleur entre le produit du bas de colonne et les buées comprimées originaires de la chlorination directe à un niveau de température de 8 à 25°C, et la température du bas de colonne étant maintenue dans la gamme de 80 à 105°C par courant adéquat de décharge du bas de colonne.

**3.** Procédé selon une des revendications précédentes,
**caractérisé en ce que** la colonne de fraction à haut point d'ébullition (9) qui fonctionne à une pression de tête de 0,7 à 1,4 bars abs. est chauffée pour assurer la purification du 1,2-dichloréthane non craqué et du 1,2-dichloréthane originaire de la colonne de déshydratation (7), le chauffage ayant lieu par un échange de chaleur entre le produit du bas de colonne et les buées comprimées originaires de la chlorination directe à une différence de température de 8 à 25°C et la température du bas de colonne étant maintenue dans la gamme de 84 à 105°C par un courant adéquat de décharge du bas de colonne.

**4.** Procédé selon une des revendications précédentes,
**caractérisé en ce que** la colonne à vide (11) utilisée pour la purification du courant de décharge du bas de la colonne à fraction de bas point d'ébullition (9) et fonctionnant à une pression de tête de 0,2 à 0,3 bars abs., est chauffée par un échange de chaleur entre le produit du bas de colonne et les buées comprimées originaires du réacteur de chlorination directe à une différence de température de 8 à 25°C, la température du bas de colonne étant maintenue dans la gamme de 80 à 90 °C par un courant adéquat de décharge du bas de colonne.

**5.** Procédé selon une des revendications précédentes,
**caractérisé en ce que** du chlore liquide utilisé pour la chlorination directe est évaporé et surchauffé par un échange de chaleur avec les buées comprimées originaires du réacteur de chlorination directe ou par un échange de chaleur avec 1,2-dichloréthane liquide circulé et originaire du réacteur de chlorination directe.

**6.** Utilisation d'un turbocompresseur pour la compression du 1,2-dichloréthane soutiré à l'état de vapeur du réacteur de chlorination directe (3), machine destinée à la réalisation d'une revendication des numéros précédents.

**7.** Utilisation d'un turbocompresseur selon la revendication No. 6, la machine étant munie d'une garniture d'arbre montée en tandem et pressurisée à l'aide de l'azote comme gaz d'obturation.

**8.** Utilisation d'un turbocompresseur selon la revendication No. 6 ou 7, **caractérisé en ce que** le débit de la machine est ajusté à la capacité du réacteur de chlorination directe moyennant un régulateur de vitesse.

Figur 1

EP 1 214 279 B1

Figur 2